(19) Europäisches Patentamt
European Patent Office
Office européen des brevets

(11) Publication number: **0 156 524 B1**

(12) **EUROPEAN PATENT SPECIFICATION**

(45) Date of publication of patent specification: **11.05.88**

(51) Int. Cl.⁴: **C 07 H 19/052,** A 61 K 31/70, C 12 P 19/38 // (C12P19/38, C12R1:04)

(21) Application number: **85301310.0**

(22) Date of filing: **27.02.85**

(54) **2'-Chloropentostatin, a pharmaceutical composition comprising the compound and a novel microorganism for producing the compound.**

(30) Priority: **16.03.84 US 590239**

(43) Date of publication of application:
**02.10.85 Bulletin 85/40**

(45) Publication of the grant of the patent:
**11.05.88 Bulletin 88/19**

(84) Designated Contracting States:
**AT BE CH DE FR GB IT LI LU NL SE**

(56) References cited:
**US-A-3 923 785**
**US-A-4 151 347**

(73) Proprietor: **WARNER-LAMBERT COMPANY**
**201 Tabor Road**
**Morris Plains New Jersey 07950 (US)**

(72) Inventor: **Schaumberg, John P.**
**1205 Westmoorland Drive**
**Ypsilanti Michigan 48197 (US)**
Inventor: **French, James C.**
**3150 Rumsey**
**Ann Arbor Michigan 48105 (US)**
Inventor: **Underhill, Marjorie A.**
**9911 Circle Drive**
**Pigeon Michigan 48755 (US)**
Inventor: **Hokanson, Gerard C.**
**2408 Antietam**
**Ann Arbor Michigan 48105 (US)**
Inventor: **Tunac, Josefino B.**
**5284 Collington Drive**
**Troy Michigan 48098 (US)**

(74) Representative: **Jones, Michael Raymond et al**
**HASELTINE LAKE & CO. Hazlitt House**
**28 Southampton Buildings Chancery Lane**
**London WC2A 1AT (GB)**

**EP 0 156 524 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The efficacy of a number of adenine nucleosides which act as both antitumor and antiviral agents is severely limited due to their rapid deactivation in vivo by the action of adenosine deaminase, an enzyme present in most mammalian body tissues. The compounds (*R*)-3-(2-deoxy-*beta*-D-erythropentofuranosyl)-3,6,7,8-tetrahydroimidazo[4,5-*d*][1,3]diazepin-8-ol (commonly known as pentostatin), disclosed in U.S. Patent 3,923,785, and its ribo-analog, (*R*)-3-(*beta-D*-ribofuranosyl)-3,6,7,8-tetrahydroimidazo[4,5-*d*][1,3]diazepin-8-ol (commonly known as coformycin), disclosed in Japanese Patent 875,639 and U.S. Patent 4,151,347, are potent inhibitors of adenosine deaminase.

2′-Chloropentostatin is a novel analog of pentostatin which is produced by an isolate of an actinomycete designated ATCC 39365.

In accordance with one aspect of the present invention, there is provided a compound having structural formula I

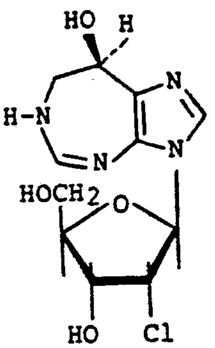

I

and the name (*R*)-3-(2-chloro-2-deoxy-*beta-D*-ribofuranosyl)-3,6,7,8-tetrahydroimidazo[4,5-*d*][1,3]-diazepin-8-ol and its pharmaceutically acceptable acid addition salts, which compound possesses potent adenosine deaminase inhibitory activity. The name 2′-chloropentostatin will be used throughout this specification to refer to the compound.

In another aspect, the present invention provides a pure strain of the 2′-chloropentostatin-producing microorganism having the identifying characteristics of isolate ATCC 39365.

In accordance with another aspect, the present invention provides a method of producing 2′-chloropentostatin by cultivating the isolate of actinomycete identified as ATCC 39365 under aerobic conditions in a medium containing assimilable sources of carbon and nitrogen until a substantial quantity of 2′-chloropentostatin is produced, and subsequently isolating the compound.

In another aspect of the present invention, there are provided pharmaceutical compositions useful for the treatment of DNA viruses comprising an effective amount of 2′-chloropentostatin or one or more of its pharmaceutically acceptable acid addition salts together with an effective amount of an antiviral agent which contains an adenine moiety such as 9-(*beta-D*-arabinofuranosyl)adenine and a pharmaceutically acceptable carrier.

The compound of the present invention may be employed in a method of treating DNA viruses in a mammal comprising administering to a mammal in need of such treatment, an effective amount of 2′-chloropentostatin or a pharmaceutically acceptable salt thereof in combination with an effective amount of 9-(*beta-D*-arabinofuranosyl)adenine together with a pharmaceutically acceptable carrier.

Brief Description of the Drawings

Figure 1 is an Ackerman-Potter plot of the adenosine deaminase inhibitory action of 2′-chloropentostatin.

Figure 2 is a plot of the ratio of velocities of uninhibited to inhibited reaction rates of adenosine with adenosine deaminase in the presence of 2′-chloropentostatin.

Figure 3 is a plot of $I_{50}$ values of 2′-chloropentostatin inhibition of adenosine deaminase versus enzyme concentration.

In accordance with the present invention, the compound 2′-chloropentostatin is produced by cultivating a selected isolate of actinomycete, designated ATCC 39365, until a substantial amount of 2′-chloropentostatin is formed, and subsequently isolating the compound.

The strain of actinomycete suitable for the purposes of this invention was found in a soil sample collected from North Carolina, USA. This microorganism was isolated from the soil sample using a suitable agar plating medium, one containing salts such as potassium phosphate, magnesium sulfate, and ferrous sulfate, and carbon sources such as glycerol and asparagine. The soil sample was plated onto the agar medium and incubated at a favorable temperature, particularly 45°C, to allow the development of the soil microorganisms.

The 2′-chloropentostatin-producing microorganism that was isolated from the agar plating medium is an as yet unidentified isolate of actinomycete and has been deposited with the American Type Culture

Collection, Rockville, Maryland 20852, where it is being maintained in their permanent collection as ATCC 39365. This organism, which produces 2′-chloropentostatin, is also being maintained as a dormant culture in lyophile tubes, cryogenic vials, and in soil tubes in the Warner-Lambert/Parke-Davis Culture Collection, 2800 Plymouth Road, Ann Arbor, Michigan 48105, where it is designated as culture WP-886.

ATCC 39365
MORPHOLOGY

| | |
|---|---|
| Aerial mycelium | Red |
| Spore chains | Flexible with a single hook at the end |
| Formation of melamine or other soluble pigments | None |
| Reduction of nitrate | Negative |
| Liquefaction of gelatin | Negative |
| Coagulation of milk | Negative |

CARBON SOURCE UTILIZATION

| | |
|---|---|
| Arabinose | Positive |
| Fructose | Positive |
| Glucose | Positive |
| Inositol | Positive/Negative |
| Mannitol | Positive |
| Raffinose | Positive |
| Rhamnose | Positive |
| Sucrose | Negative |
| Xylose | Positive |
| Salicin | Negative |
| Galactose | Negative |

The compound 2′-chloropentostatin, which exhibits potent activity as an inhibitor of adenosine deaminase, is produced by isolate ATCC 39365 during aerobic fermentation under controlled conditions. The fermentation medium consists of sources of carbon, nitrogen, minerals, and growth factors. Examples of suitable carbon sources include glycerol and various simple sugars such as glucose, mannose, fructose, xylose, ribose, or other carbohydrate-containing compounds such as dextrin, starch, corn meal, and whey. The normal quantity of carbon source materials in the fermentation medium varies from about 0.1 to about 10 weight percent.

Nitrogen sources in the fermentation medium are inorganic, organic, and mixed inorganic-organic nitrogenous materials. Examples of such materials are cottonseed meals, soybean meal, corn germ flour, corn steep liquor, distiller's dried solubles, peanut meal, peptonized milk, and various ammonium salts.

The addition of minerals and growth factors to the fermentation medium is also helpful in the production of 2′-chloropentostatin. Examples of such mineral additives include sodium chloride, potassium chloride, ferrous sulfate, calcium carbonate, cobalt chloride, and zinc sulfate. Sources of growth factors include various yeast and milk products.

The preferred method of producing 2′-chloropentostatin is by submerged culture fermentation. According to this method, the fermentation medium ingredients are prepared in solution or suspension in water, and the mixture is subsequently sterilized by autoclaving or steam heating. The mixture is cooled following sterilization to a temperature between about 16°C and 45°C and the pH is adjusted to preferably

between about pH 4 and about pH 8. The cooled, sterile medium is inoculated with the organism and thereafter fermentation is carried out with aeration and agitation.

In the submerged culture method, fermentation is carried out in shake-flasks or in stationary tank fermentors. In shake-flasks, aeration is effected by agitating the flask and contents to bring about contact of the medium with air. In stationary tank fermentors, agitation is provided by impellers which may take the form of disc turbines, vaned discs, open turbine or marine propellers. Aeration is accomplished by sparging air or oxygen into the agitated mixture. Adequate production of 2′-chloropentostatin is achieved under these conditions after a period of about two to ten days.

Alternatively, 2′-chloropentostatin may be produced by solid state fermentation of the microorganism.

The following examples are provided to illustrate the fermentative production of 2′-chloropentostatin. The examples are merely illustrative and are not to be read as limiting the scope of the invention as it is defined by the appended claims.

Fermentative Production of 2′-Chloropentostatin Shake-Flask Fermentation

Example 1

The ATCC 39365 culture, following its isolation by the agar plating technique, was transferred from its dormant state to an agar slant tube containing CIM 23 agar medium and incubated at 28°C for 7-14 days. A portion of the microbial growth which developed in this slant tube was used to inoculate 5 ml of SD-05 seed medium contained in a 18 × 150 mm tube. The tube contents were shaken on a gyratory shaker at 170 rpm and incubated at 33°C for four days.

TABLE 1
Formulation of CIM 23 Agar Medium

| | |
|---|---|
| Amidex corn starch | 10 g |
| N-Z Amine, Type A | 2 g |
| Beef extract (Difco) | 1 g |
| Yeast extract (Difco) | 1 g |
| Cobaltous chloride $5H_2O$ | 20 mg |
| Agar | 20 g |
| Distilled Water | 1000 ml |

TABLE 2
Formulation of SD-05 Seed Medium

| | |
|---|---|
| Amberex 1003 (Amberex Laboratories) | 5 g |
| Glucose monohydrate | 1 g |
| Dextrin-Amidex B411 (Corn Products) | 24 g |
| N-Z Case (Humko Sheffield) | 5 g |
| Spray-dried meat solubles (Daylin) | 3 g |
| Calcium carbonate | 2 g |
| Water | 1000 ml |

Example 2

A 1.0-ml portion of the contents of the seed tube from Example 1 was transferred to a 300-ml shake-flask containing 50 ml of SM-31 screening medium. The inoculated flask contents were incubated at 33°C for four days with shaking (170 rpm gyratory shaking, 5 cm throw).

TABLE 3
Formulation of SM-31 Screening Medium

| | |
|---|---|
| Glucose monohydrate | 15 g |
| Lactose | 10 g |
| Distiller's solubles | 6.5 g |
| Peptonized milk | 3.5 g |
| Torula yeast | 2.5 g |
| Water, pH adjusted to 7.0 | 1000 ml |

To confirm the fermentation activity of the microorganism, a second microbial seed was prepared as described in Example 2 above and 2 ml of this seed was used to inoculate 50 ml of SM-31 screening medium contained in a 300-ml shake-flask. The inoculated flask was incubated at 300°C for four days with shaking (170 rpm gyratory shaking, 5 cm throw).

Example 3

The production of 2′-chloropentostatin in the fermentation beer of Example 2 was monitored by screening the beer against the microorganism *Streptococcus faecalis* 05045. Agar plates containing AM-10 assay medium were inoculated with this microorganism and paper discs (12.7 mm diameter), impregnated with the fermentation beer, were placed on the inoculated agar medium and incubated overnight at 37°C. The diameter of the zones of inhibition around each disc were measured. The size of the zone correlated with the amount of 2′-chloropentostatin present in the fermentation beer. The results of these measurements appear in Table 5.

TABLE 4
Formulation of AM-10 Assay Medium

| | | |
|---|---|---|
| $K_2HPO_4$ | 3.9 | g |
| Dextrose | 25.0 | g |
| Sodium citrate · 2 $H_2O$ | 34.4 | g |
| Casein hydrolysate | 6.2 | g |
| Asparagine | 375 | mg |
| L-tryptophan | 125 | mg |
| Cysteine | 312.5 | mg |
| Glutathione | 0.31 | mg |
| Thiamine HCl | 250 | µg |
| Riboflavin | 625 | µg |
| Ca pantothenate | 500 | µg |
| Nicotinic acid | 500 | µg |
| p-aminobenzoic acid | 625 | µg |
| Biotin | 12.5 | µg |
| Folic acid | 500 | µg |
| Pyridoxine HCl | 2.5 | mg |
| NaCl | 12.5 | mg |
| $MgSO_4$ | 250 | mg |
| $FeSO_4$ | 12.5 | mg |
| $MnSO_4 \cdot H_2O$ | 125.0 | mg |
| Tween 80 | 62.5 | mg |
| Adenine sulfate | 6.25 | mg |
| Agar | 15.0 | g |
| Water | 1000 | ml |

TABLE 5
Initial Activity of Shake-Flask Fermentation Beers
vs. *Streptococcus faecalis* 05045

| Fermentation Stage | Activity (zone diameter, mm) |
|---|---|
| Shake-flask I | 48 |
| Shake-flask II | 54 |

The crude fermentation beer also demonstrated antimicrobial activity against the microorganisms *Branhamella catarrhalis* 03596 and *Escherichia coli* 05117.

Large Batch Fermentation

Example 4

A cryogenically preserved culture sample of ATCC 39365 was thawed and used to inoculate 600 ml of SD-05 seed medium contained in a 2-liter baffled Erlenmeyer flask. The flask and contents were incubated for 70 hours at 33°C with shaking (130 rpm, 5 cm throw).

The contents of this shake-flask were used to inoculate 16 liters of SD-05 seed medium contained in a 30-liter stirred-jar fermentor. The jar contents were incubated at 33°C for 24 hours with stirring at 300 rpm. During the incubation period, the jar contents were sparged with air at a rate of 1 volume air/volume medium/minute.

Example 5

The contents of the stirred-jar of Example 4 were used to inoculate 160 gallons (605.7 liters) of SM-31 fermentation medium contained in a 200 gallon (757.1 liter) fermentation tank. The fermentation medium was sterilized by steam heating for 40 minutes at 121°C and then cooled to 33°C. The cooled fermentation medium was inoculated with about 15 liters of the seed from Example 4 and allowed to ferment for five days at 33°C with stirring at 155 rpm. The stirred tank contents were sparged with air at a rate of about 1 volume air/volume medium/minute during the fermentation. Antifoam P-2000 was used to control foaming as needed.

The production of 2′-chloropentostatin was monitored throughout the process by the assay described in detail above in Example 3 and the pH and percent growth, measured as sedimentation values, were recorded. The results of these tests appear in Table 6.

TABLE 6

| Fermentation time (Hrs) | pH | % Growth (sedimentation values) | Zone Diameter (mm) |
|---|---|---|---|
| 0 | 5.95 | — | — |
| 12 | 6.2 | 4.0 | — |
| 24 | 6.7 | 5.3 | — |
| 36 | 7.3 | 10.0 | — |
| 48 | 7.0 | 11.3 | — |
| 71 | 7.0 | 16.7 | — |
| 96 | 6.9 | 18.7 | — |
| 119 | 6.85 | 20.7 | 48 |

Example 6

A cryogenically preserved 1-ml sample of culture ATCC 39365 was thawed and aseptically transferred to a 2-liter baffled Erlenmeyer flask containing 600 ml of sterile SD-05 seed medium. The inoculated flask contents were incubated at 33°C for 71 hours with shaking (130 rpm, 5 cm throw).

After 71 hours, the contents of the flask were aseptically transferred to 16 liters of sterile SD-05 seed medium contained in a 30-liter stirred-jar fermentor. This inoculum was incubated at 33°C for 22 hours with stirring at 300 rpm and sparging with air at a rate of 1 volume air/volume medium/minute.

Example 7

SM-19 medium (300 gallon, 1135.6 liters), contained in a 500-gallon (1892.7 liter) fermentation tank were sterilized by heating with steam at 121°C for 40 minutes. The medium was cooled to 33°C and then inoculated with 30 liters of inoculum prepared as described in Example 6. The inoculated medium was allowed to ferment for five days at 33°C with stirring at 84 rpm and sparging with air at a rate of 0.375 volume air/volume median/minute. Dow-Corning "C" antifoam agent was used to control foaming as needed.

TABLE 7
Formulation of SM-18 Fermentation Medium

| | |
|---|---|
| Dextrin | 1.5 % |
| Lactose | 1.0 % |
| Distiller's solubles | 0.65% |
| Peptonized milk | 0.35% |
| Torula yeast | 0.25% |
| Tap water | 100.0 % |
| pH adjusted to 7.0 with NaOH | |

The production of 2′-chloropentostatin was monitored throughout the fermentation cycle using *Streptococcus faecalis* 05045 as described in Example 3. Additional fermentation parameters such as pH and percent sedimentation were also recorded. The results of these observations appear in Table 8.

TABLE 8
Fermentation in a 500-Gallon (1892.7 liters) Tank

| Fermentation time (hrs) | pH | % Growth (sedimentation values) | Zone Diameter (mm)* |
|---|---|---|---|
| 0 | 6.3 | — | — |
| 12 | 6.55 | 4.7 | — |
| 24 | 7.20 | 10.0 | 42 |
| 36 | 7.30 | 10.0 | 46 |
| 48 | 7.20 | 10.7 | 48 |
| 60 | 7.35 | 16.7 | 50 |
| 72 | 7.20 | 30.0 | 52.5 |
| 88 | 7.30 | 50.0 | 53.5 |
| 96 | 7.40 | 56.0 | 53.5 |
| 112 | 6.90 | 59.9 | 54.0 |
| 120 | 7.25 | 96.6 | 54.0 |

* Measured activity against *Streptococcus faecalis* 05045.

Chemical Isolation of 2′-Chloropentostatin

A 680-liter portion of the harvested beer from Example 7 was adjusted to pH 6.5 and mixed with 31 kg of Celite® 545 and filtered through a plate and frame filter press. The filtrate (680 liters) was mixed with 30 kg (4.4% w/v) Darco® G-60 and, after the addition of 15.5 kg of Celite® 545, filtered once more through a clean plate and frame filter press. The filter cake was washed with deionized water (185 liters), then eluted by circulating acetone-water (1:1, 151 liters) through the press three times. The acetone-water eluates, which contained most of the 2′-chloropentostatin, were combined and concentrated to 21 liters.

An eighteen-liter portion of the above 21 liter concentrate was stirred with 500 grams of Celite® 545 and then filtered. Following the adjustment of pH from 6.5 to 5.1, the resulting filtrate (17 liters) was passed over ten liters of Dowex®-50 x 2 resin (hydrogen form). After washing the resin with deionized water (19 liters),

the column was eluted with 1N ammonium hydroxide (42 liters). The ammonium hydroxide eluate, which contained all of the 2′-chloropentostatin (as determined by HPLC assay), was concentrated to 400 milliliters and passed over 10 liters of Sephadex® G-10. The column was eluted with deionized water, and nine 0.5-liter fractions and seven 1-liter fractions were collected. Most of the 2′-chloropentostatin was present in fractions fifteen and sixteen, each of which was concentrated to 200 ml and lyophilized to yield 6.3 g and 6.0 g, respectively, of amorphous solid. The 6.3 g of solid from fraction fifteen was treated with hot absolute ethanol (50 ml) affording 3.86 g of crystalline 2′-chloropentostatin upon cooling. Recrystallization from water (35 ml) yielded 2.9 g of colorless needles. Similar treatment of the 6.0 g of solid from fraction sixteen afforded 1.55 g of recrystallized 2′-chloropentostatin.

Properties of 2′-Chloropentostatin

Melting Point:
decomposition at approximately 180°C

Ultraviolet Absorption Spectrum

| | |
|---|---|
| $\lambda_{max}$ (ε), Methanol | 284 nm (9380) |
| $\lambda_{max}$ (ε), 0.05 M methanolic HCL | 266 nm (8473) |

Optical Rotation
$[\alpha]_D^{23}$ + 28.5° (1.26% in 0.1 M pH 7 phosphate buffer)

| Elemental Analysis | %C | %H | %Gl | %N |
|---|---|---|---|---|
| Calcd. for $C_{11}H_{15}ClN_4O_4$ | 43.64 | 4.99 | 11.75 | 18.51 |
| Found | 43.83 | 4.96 | 11.76 | 18.62 |

Mass Spectrum (via fast atom bombardment)

| | |
|---|---|
| Calcd. for $C_{11}H_{16}ClN_4O_4$ [M + H] | 303.0860 m/z |
| Found | 303.0868 m/z |

Infrared Absorption Spectra in KBr
Principal absorptions at 3350, 1635, 1625, 1198, 1100, 1065, and 1048 reciprocal centimeters.

360 MHz Proton Magnetic Resonance Spectrum in $D_2O$ Principal signals at:
(S = singlet, d = doublet, dd = doublet of doublets, m = multiplet) 3.26 m (2H), 3.65 m (2H), 4.11 m (1H), 4.33 dd (1H), 4.71 dd (1H), 4.98 d (1H), 5.87 d (1H), 7.01 s (1H), and 7.55 s (1H) parts per million downfield from sodium 2,2-dimethyl-2-silapentane-5-sulfonate (DSS).

90.4 MHz Carbon-13 Magnetic Resonance Spectrum in $D_2O$ Principal signals at:

| Peak number | Chemical shift* |
|---|---|
| 1 | 152.9 |
| 2 | 138.1 |
| 3 | 134.8 |
| 4 | 131.6 |
| 5 | 90.8 |
| 6 | 87.9 |
| 7 | 73.3 |
| 8 | 69.5 |
| 9 | 63.9 |
| 10 | 63.5 |
| 11 | 49.9 |

* Parts per million downfield from tetramethylsilane.

High Pressure Liquid Chromatography

| | |
|---|---|
| Column: | µBondapak® $C_{18}$ silica gel (3.9 mm I.D. x 30 cm) |
| Solvent: | 0.02 M pH 7.0 sodium phosphate buffer-acetonitrile (90:10) |
| Flow rate: | 1.0 ml/min |
| Detection: | ultraviolet absorption at 280 nm |
| Retention time: | 5.1 minutes. |

Thin Layer Chromatography on Silica Gel 60 F254 (E. Merck)

| | |
|---|---|
| Solvent: | chloroform-ethanol-0.5 M sodium acetate pH 5.5 (40:70:20) |
| Detection: | iodine vapor |
| $R_f$: | 0.51 |

The compound of the invention forms pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, malonic, ascorbic, maleic, methanesulfonic and the like. The salts are prepared by contacting the free base form with an equivalent amount of the desired acid in the conventional manner. The free base form may be regenerated by treating the salt form with a base. For example, dilute aqueous base solutions may be utilized. Dilute aqueous sodium hydroxide, potassium carbonate, ammonia and sodium bicarbonate solutions are suitable for this purpose. The free base form differs from its respective salt form somewhat in certain physical properties such as solubility in polar solvents, but the salts are otherwise equivalent to the free base form for purposes of the invention.

Antimicrobial Activity

Paper disks (12.7 mm in diameter) impregnated with an aqueous solution containing varying amounts of 2'-chloropentostatin were placed on a layer of agar containing AM-10 assay medium (Table 4) and inoculated with *Streptococcus faecalis* 05045. After incubation at 37° overnight the following zones of inhibition were observed:

| Concentration | Zone diameter |
|---|---|
| 2000 µg/ml | 64 mm |
| 200 µg/ml | 61 mm |
| 20 µg/ml | 55 mm |
| 2 µg/ml | 47 mm |
| 0.2 µg/ml | 40 mm |

Adenosine Deaminase Activity of 2'-Chloropentostatin

The potency of 2'-chloropentostatin as an adenosine deaminase inhibitor was determined using the following methods. The velocity (in moles/minute/ml) of the reaction of adenosine deaminase with adenosine was measured for several concentrations of the enzyme in the presence of various concentrations of the inhibitor, 2'-chloropentostatin. The data has been plotted in Figure 1 in the form of an Ackerman-Potter plot (see W. W. Ackerman and V. R. Potter, *Proc. Soc. Exp. Biol. Med., 72*:1 (1949)). The fact that the traces, at higher concentrations of substrate, become parallel is indicative of the fact that 2'-chloropentostatin is a so-called "tight-binding" or "pseudo-irreversible" inhibitor of the enzyme, adenosine deaminase.

In Figure 2, the ratio of the velocity of the uninhibited reaction of adenosine with adenosine deaminase to the velocity of the same reaction in the presence of the inhibitor, 2'-chloropentostatin, has been plotted for several concentrations of substrate versus the micromolar concentrations of 2'-chloropentostatin. The values of $V_o/V_i = 2$ for each curve yield the $I_{50}$ for the enzyme inhibitor at each substrate concentration. These $I_{50}$ values have been plotted versus substrate concentration in Figure 3. Extrapolation of the linear plot of Figure 3 permits the determination of $Ki = 1 \times 10^{-10}$ molar for 2'-chloropentostatin.

The compound of the present invention, 2'-chloropentostatin is a very useful substance not only for the analysis of the causes of disease involving metabolism of adenosine and nucleic acids, but also as an agent for potentiating the activity of antiviral agents which contain an adenine moiety such as the substance 9-

(*beta-D*-arabinofuranosyl)adenine. The latter substance is disclosed in U.S. Patent 3,616,208 (incorporated herein by reference) as a useful agent for the treatment of DNA viruses, especially herpes and vaccinia viruses in mammals. The biological efficacy of adenine-derived antiviral agents such as 9-(*beta-D*-arabinofuranosyl)adenine, is greatly diminished by the rapid deamination of such materials in vivo by the enzyme, adenosine deaminase.

2′-Chloropentostatin is administered in conjunction with adenine-derived antiviral agents to potentiate the activity of the latter by inhibiting adenosine deaminase enzymes. In a preferred embodiment, 2′-chloropentostatin is combined in a pharmaceutical composition with the antiviral agent 9-(*beta-D*-arabinofuranosyl)adenine.

More particularly, 2′-chloropentostatin is administered in combination with an adenine-derived antiviral agent in ratios of from about 0.005 to about 0.5 parts of 2′-chloropentostatin to about 1 part of the antiviral agent. The preferred range is from 0.01 to 0.25 parts of 2′-chloropentostatin to 1 part of the antiviral agent. In the particular case where 2′-chloropentostatin is administered together with 9-(*beta-D*-arabinofuranosyl)adenine, the preferred range is from 0.01 to 0.25 parts of the compound of this invention to 1 part of 9-(*beta-D*-arabinofuranosyl)adenine. More specifically, when the composition is administered parenterally, preferably intravenously, injectable solutions are given so as to provide the host with from 0.1 mg to 5.0 mg of 9-(*beta-D*-arabinofuranosyl)adenine per kg of body weight and 0.0005 mg to 0.1 mg of the compound of this invention per kg of body weight per day. The preferred quantity which is administered on a daily basis is from about 0.5 mg to 5.0 mg of 9-(*beta-D*-arabinofuranosyl)adenine per kg of body weight to about .005 mg to 0.02 mg of the compound of this invention per kg of body weight.

The pharmaceutical composition may be in bulk form containing 0.005 to 0.5 parts of the compound of this invention to about 1 part of 9-(*beta-D*-arabinofuranosyl)adenine which is placed in solution at time of use by the addition of a solvent which is appropriate for injectables. In the alternative, the pharmaceutical composition may be an aqueous solution containing a ratio of from 0.005 to 0.5 parts of the compound of this invention to about 1 part of 9-(*beta-D*-arabinofuranosyl)adenine and other materials such as preservatives, buffering agents, agents intended to adjust the isotonicity of the solution, etc. The volume of water is not critical and may vary from less than 1 ml to about 500 ml.

**Claims for the Contracting States: BE CH DE FR GB IT LI LU NL SE**

1. The compound 2′-chloropentostatin having adenosine deaminase inhibitory activity and possessing the structure

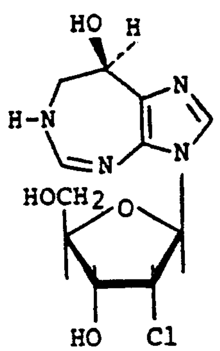

and its pharmaceutically acceptable acid addition salts.

2. A pharmaceutical composition, useful for the treatment of DNA viruses, comprising an effective amount of a compound selected from 2′-chloropentostatin and its pharmaceutically acceptable acid addition salts in combination with an effective amount of an antiviral agent containing an adenine moiety and a pharmaceutically acceptable carrier or diluent.

3. A pharmaceutical composition in accordance with claim 2 wherein said antiviral agent is 9-(*beta-D*-arabinofuranosyl)adenine.

4. A process for the production of 2′-chloropentostatin which comprises cultivating a strain of actinomycete, identified as ATCC 39365, under aerobic conditions in a culture medium containing assimilable sources of carbon and nitrogen until a substantial amount of 2′-chloropentostatin is produced and subsequently isolating said 2′-chloropentostatin compound.

5. A purified isolate of an actinomycete having the identifying characteristics of ATCC 39365, which isolate is capable of producing 2′-chloropentostatin under conditions of aerobic fermentation in a culture medium containing assimilable sources of carbon and nitrogen.

**Claims for the Contracting State: AT**

1. A process for the production of 2′-chloropentostatin having the structure

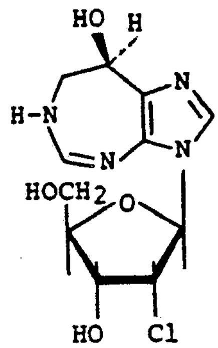

and its pharmaceutically acceptable acid addition salts, which process comprises cultivating a strain of actinomycete, identified as ATCC 39365, under aerobic conditions in a culture medium containing assimilable sources of carbon and nitrogen until a substantial amount of 2′-chloropentostatin is produced and subsequently isolating said 2′-chloropentostatin compound; and optionally forming a pharmaceutically acceptable acid addition salt thereof.

2. A process for preparing a pharmaceutical composition useful for the treatment of DNA viruses, which process comprises combining an effective amount of 2′-chloropentostatin or a pharmaceutically acceptable acid addition salt thereof prepared by a process as claimed in Claim 1, together with an effective amount of an antiviral agent containing an adenine moiety and a pharmaceutically acceptable carrier or diluent.

3. A process according to Claim 2, wherein said antiviral agent is 9-(*beta-D*-arabinofuranosyl)adenine.

4. A purified isolate of an actinomycete having the identifying characteristics of ATCC 39365, which isolate is capable of producing 2′-chloropentostatin under conditions of aerobic fermentation in a culture medium containing assimilable sources of carbon and nitrogen.

**Patentansprüche für die Vertragsstaaten: BE CH DE FR GB IT LI LU NL SE**

1. Die Verbindung 2′-Chloropentostatin mit Adenosindeaminase-hemmender Aktivität, die die Struktur

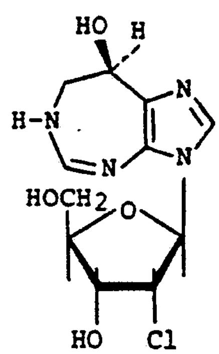

besitzt, und deren pharmazeutisch akzeptable Säureadditionssalze.

2. Eine pharmazeutische Zusammensetzung, die für die Behandlung von DNR-Viren geeignet ist, umfassend eine wirksame Menge einer Verbindung, die aus 2′-Chlorpentostatin und seinen pharmazeutisch akzeptablen Säureadditionssalzen ausgewählt ist, in Kombination mit einer wirksamen Menge eines antiviralen Mittels, das einen Adeninanteil und ein pharmazeutisch akzeptables Träger- oder Verdünnungsmittel enthält.

3. Eine pharmazeutische Zusammensetzung gemäß Anspruch 2, worin dieses antivirale Mittel 9-(*beta-D*-Arabinofuranosyl)-adenin ist.

4. Verfahren zur Herstellung von 2′-Chloropentostatin, welches das Züchten eines Stammes von Actinomyceten, als ATCC 39365 identifiziert, unter aeroben Bedingungen in einem assimilierbare Kohlenstoff- und Stickstoffquellen enthaltenden Kulturmedium umfaßt, bis eine wesentliche Menge an 2′-Chloropentostatin produziert worden ist, und das nachfolgende Isolierenden dieser 2′-Chloropentostatinverbindung.

5. Ein gereinigtes Isolat eines Actinomyceten mit den Identifikationscharakteristika von ATCC 39365, welches Isolat die Fähigkeit besitzt, unter Bedingungen aerober Fermentation in einem assimilierbare Kohlenstoff- und Stickstoffquellen enthaltenden Kulturmedium 2′-Chloropentostatin zu erzeugen.

**Patentansprüche für den Vertragsstaat: AT**

1. Ein Verfahren zur Herstellung von 2′-Chloropentostatin mit der Struktur

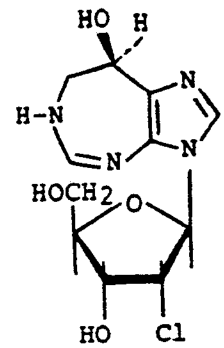

und dessen pharmazeutisch akzeptablen Säureadditionssalzen, welches Verfahren das Züchten eines Stammes von Actinomyceten, als ATCC 39365 identifiziert, unter aeroben Bedingungen in einem assimilierbare Kohlenstoff- und Stickstoffquellen enthaltenden Kulturmedium umfaßt, bis eine wesentliche Menge an 2′-Chlorpentostatin produziert worden ist, und das nachfolgende Isolieren dieser 2′-Chlorpentostatinverbindung; und gegebenenfalls die Bildung eines pharmazeutisch akzeptablen Säureadditionssalzes davon.

2. Ein Verfahren zur Herstellung einer pharmazeutischen Zusammensetzung, die für die Behandlung von DNR-Viren geeignet ist, welches Verfahren die Kombination einer wirksamen Menge von 2′-chloropentostatin oder einem pharmazeutisch akzeptablen Säureadditionssalz davon, hergestellt mittels eines Verfahrens, wie in Anspruch 1 beansprucht, zusammen mit einer wirksamen Menge eines antiviralen Mittels, das einen Adeninanteil und ein pharmazeutisch akzeptables Träger- oder Verdünnungsmittel enthält, umfaßt.

3. Varfahren nach Anspruch 2, worin das antivirale Mittel 9-(*beta-D*-Arabinofuranosyl)-adenin ist.

4. Ein gereinigtes Isolat eines Actinomyces mit den Identifikationscharakteristika von ATCC 39365, welches Isolat die Fähigkeit besitzt, unter Bedingungen aerober Fermentation in einem assimilierbare Kohlenstoff- und Stickstoffquellen enthaltenden Kulturmedium 2′-Chloropentostatin zu erzeugen.

**Revendications pour les Etats contractants: BE CH DE FR GB IT LI LU NL SE**

1. Le dérivé, la 2′-chloropentostatine, présentant une activité inhibitrice de l'adénosine déaminase et présentant la structure:

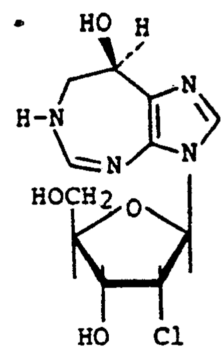

et ses sels d'addition d'acides pharmaceutiquement acceptables.

2. Une composition pharmaceutique utile au traitement des virus à DNA comprenant une quantité efficace d'un composé consistant en 2′-chloropentostatine ou en ses sels d'addition d'acides pharmaceutiquement acceptables, en association avec une quantité efficace d'un agent antiviral contenant un radical adénine et comprenant un support ou diluant pharmaceutiquement acceptable.

3. Une composition pharmaceutique selon la revendication 2, dans laquelle cet agent antiviral est la 9-(béta-D-arabinofuranosyl)adénine.

4. Un procédé de préparation de la 2'-chloropentostatine qui consiste à cultiver une souche d'actinomycète identifiée sous le numéro ATCC 39365 dans des conditions aérobies dans un milieu de culture contenant des sources assimilable de carbone et d'azote jusqu'à ce que se forme une quantité substantielle de 2'-chloropentostatine et ensuite à séparer ce dérivé, la 2'-chloropentostatine.

5. Un isolat purifié d'un actinomycète présentant les caractéristiques d'identification de l'ATCC 39365, cet isolat étant capable de donner lieu à la formation de 2'-chloropentostatine dans des conditions de fermentation aérobie dans un milieu de culture contenant des sources assimilables de carbone et d'azote.

**Revendications pour l'Etat contractant: AT**

1. Un procédé de préparation de 2'-chloropentostatine de structure:

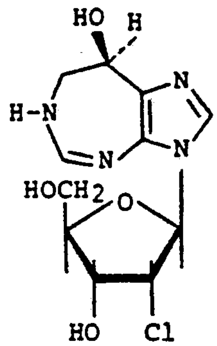

et de ses sels d'addition d'acides pharmaceutiquement acceptables, ce procédé consistant à cultiver une souche d'actinomycète identifiée sous le numéro ATCC 39365 dans des conditions aérobies dans un milieu de culture contenant des sources assimilable de carbone et d'azote jusqu'à ce que se forme une quantité substantielle de 2'-chloropentostatine et ensuite à isoler ce dérivé, la 2'-chloropentostatine; et éventuellement à en former un sel d'addition d'acide pharmaceutiquement acceptable.

2. Un procédé de préparation d'une composition pharmaceutique utile au traitement des virus à DNA, ce procédé consistant à associer une quantité efficace de 2'-chloropentostatine ou d'un sel d'addition d'acide pharmaceutiquement acceptable préparée par un procédé selon la revendication 1, à une quantité efficace d'un agent antiviral contenant un radical adénine et à un support ou diluant pharmaceutiquement acceptable.

3. Un procédé selon la revendication 2, dans lequel cet agent antiviral est la 9-(béta-D-arabinofuranosyl)adénine.

4. Un isolat purifié d'un actinomycète présentant les caractéristiques d'identification de l'ATCC 39365, cet isolat étant capable de donner lieu à la formation de 2'-chloropentostatine dans des conditions de fermentation aérobie dans un milieu de culture contenant des sources assimilables de carbone et d'azote.

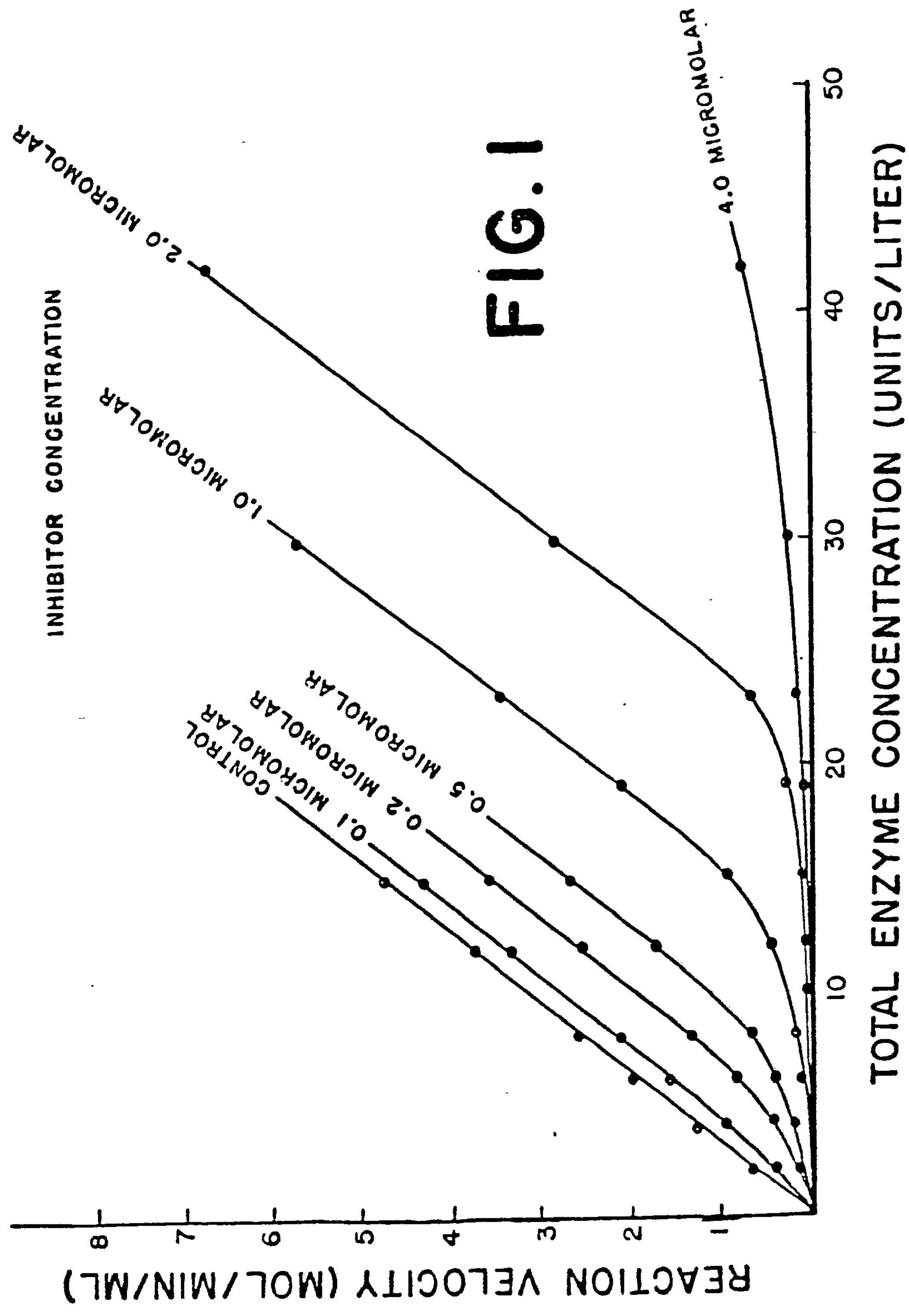
INHIBITOR CONCENTRATION
CONTROL
0.1 MICROMOLAR
0.2 MICROMOLAR
0.5 MICROMOLAR
1.0 MICROMOLAR
2.0 MICROMOLAR
4.0 MICROMOLAR
REACTION VELOCITY (MOL/MIN/ML)
TOTAL ENZYME CONCENTRATION (UNITS/LITER)
1
2
3
4
5
6
7
8
10
20
30
40
50

FIG. I

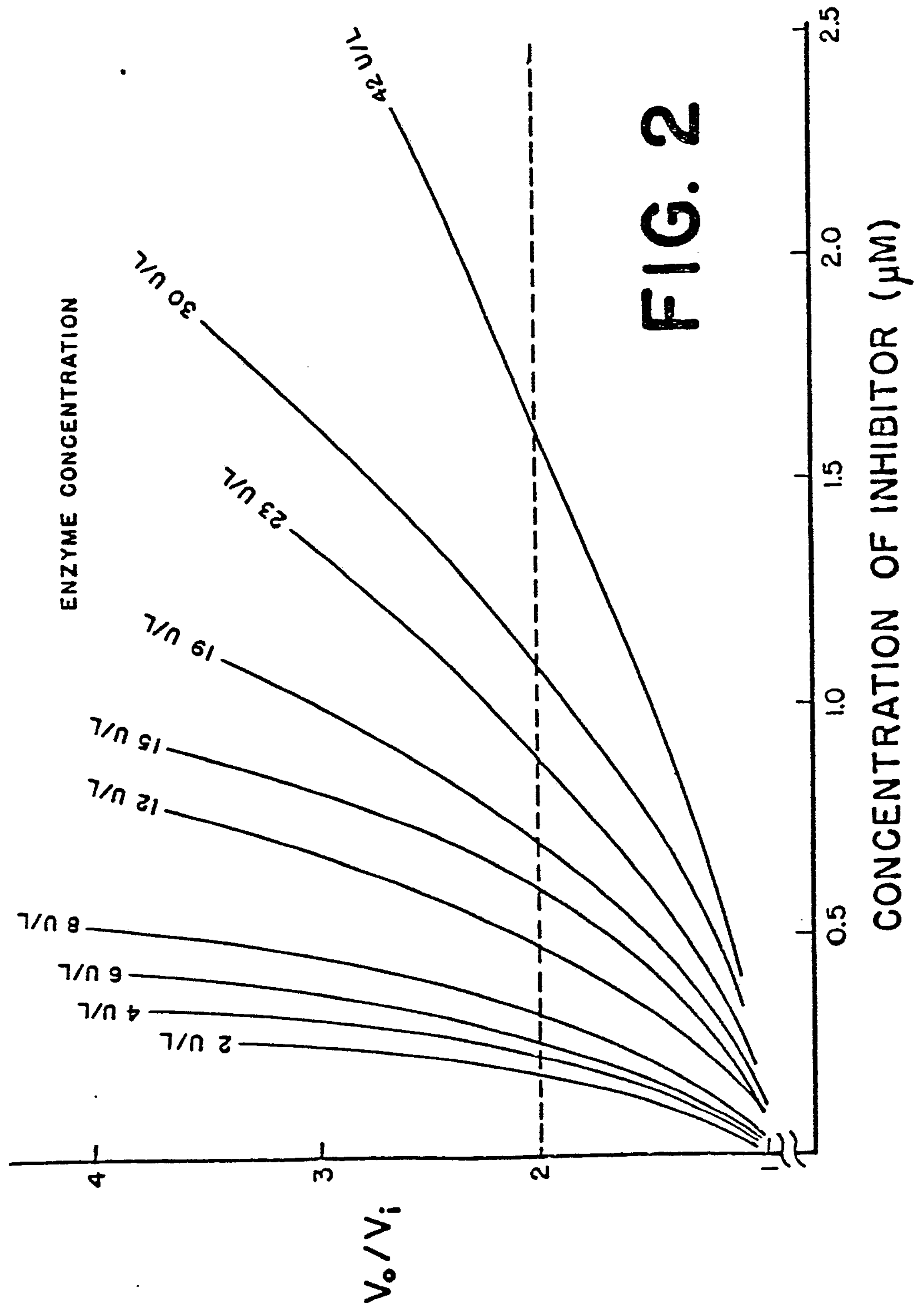
ENZYME CONCENTRATION
2 U/L
4 U/L
6 U/L
8 U/L
12 U/L
15 U/L
19 U/L
23 U/L
30 U/L
42 U/L
$V_o/V_i$
1
2
3
4
0.5
1.0
1.5
2.0
2.5
CONCENTRATION OF INHIBITOR (μM)

FIG. 2

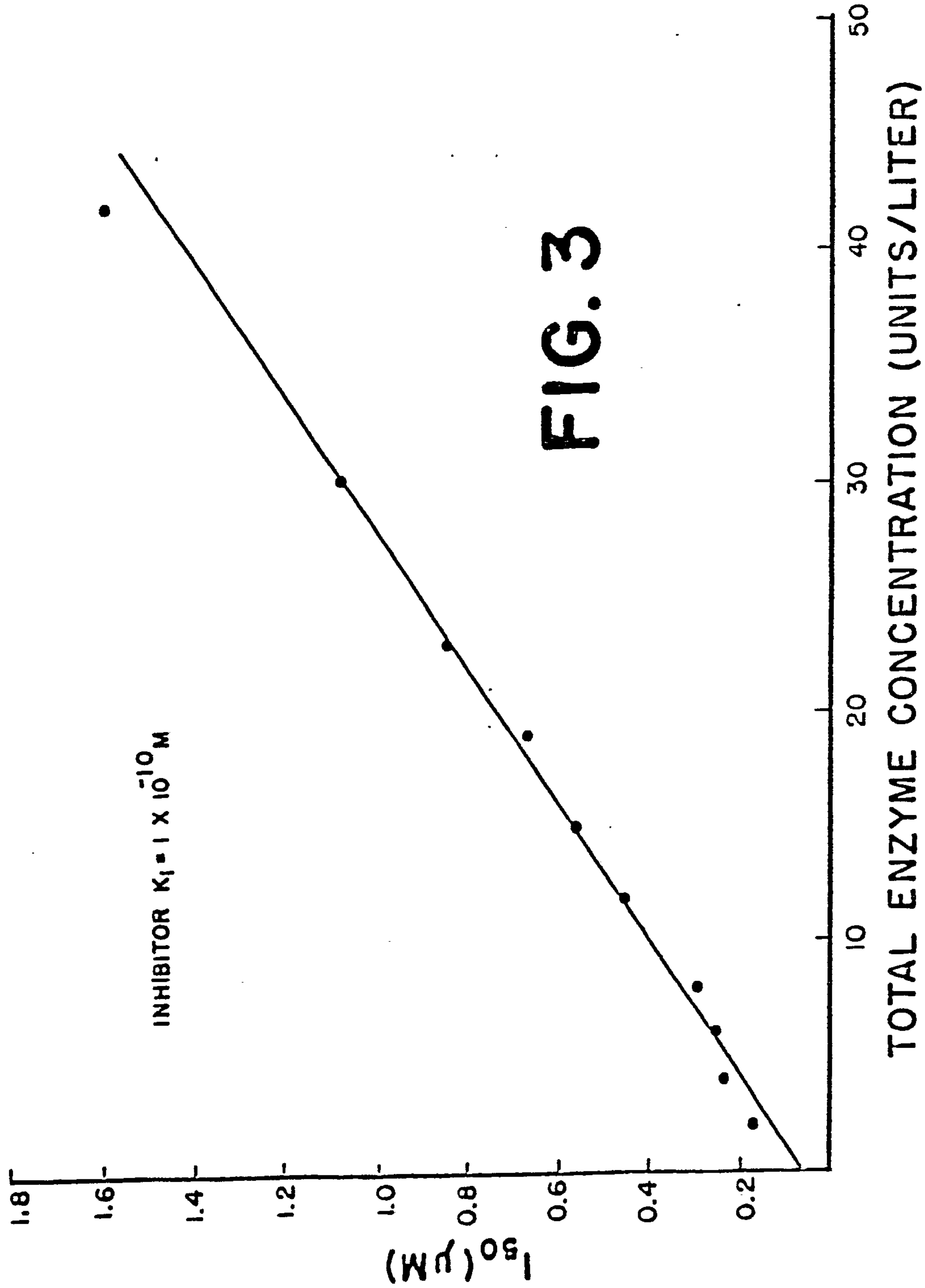
INHIBITOR $K_I = 1 \times 10^{-10}$ M
$I_{50}$ (µM)
1.8
1.6
1.4
1.2
1.0
0.8
0.6
0.4
0.2
10
20
30
40
50
TOTAL ENZYME CONCENTRATION (UNITS/LITER)

FIG. 3